# EUROPÄISCHE PATENTSCHRIFT

(11) **EP 2 008 606 B1**
(45) Veröffentlichungstag und Bekanntmachung des Hinweises auf die Patenterteilung: **05.08.2009**
(21) Anmeldenummer: 07111415.1
(22) Anmeldetag: 29.06.2007
(51) Int. Cl.: A61B 19/00, A61B 17/00

(54) **Bestimmung von Korrespondenz-Objekt-Paaren zur medizinischen Navigation**
Determination of correspondence object pairs for medical navigation
Détermination de la correspondance entre un objet et une paire pour la navigation médicale

(43) Veröffentlichungstag der Anmeldung: 31.12.2008
(73) Patentinhaber: BrainLAB AG, 85622 Feldkirchen (DE)
(72) Erfinder: Haimerl, Martin, 82205 Gilching (DE); Fleig, Oliver, 85598, Baldham (DE); Grünschläger, Frank, 85622, Feldkirchen (DE); Hepke, Markus, 80538, München (DE); Haberl, Martin, 81373, München (DE)
(74) Vertreter: Schwabe - Sandmair - Marx

(56) Entgegenhaltungen:
- WO-A-20/06048651
- WO-A-20/06106335
- US-A- 6 096 050
- US-A1- 2002 147 455

## Beschreibung

Die vorliegende Erfindung betrifft die Unterstützung der Bestimmung von Korrespondenz-Objekt-Paaren, die dieselben Objekte der selben anatomischen Körperstruktur in verschiedenen Bezugssystemen darstellen. Die bestimmten Korrespondenz-Objekt-Paare können verwendet werden, um eine Navigation relativ zu einer anatomischen Körperstruktur zu ermöglichen.

Ein Korrespondenz-Objekt-Paar beschreibt dasselbe Objekt einer anatomischen Körperstruktur in zwei verschiedenen Bezugssystemen. Üblicherweise werden so genannte anatomische Landmarken als Objekte verwendet, da sie in der anatomischen Körperstruktur für den Arzt leicht zu identifizieren sind. Erfindungsgemäß können nicht nur diese Landmarken sondern jegliche anderen Objekte wie z.B. Teile der anatomischen Struktur oder geometrische Primitive, die eine spezielle Beziehung zu der Körperstruktur haben (z.B. Punkte auf der Oberfläche, Symmetrieobjekte, Tangentialobjekte, eingepasste geometrische Objekte, verwendet werden, um Korrespondenz-Objekt-Paare zu bestimmen, und um insbesondere die räumliche Beziehung zwischen den beiden Bezugssystemen zu bestimmen. Letzteres ist die Voraussetzung, um eine Navigation relativ zur Körperstruktur durchzuführen.

Üblicherweise gibt es ein (virtuelles) Bezugssystem, in dem eine dreidimensionale räumliche Beschreibungen einer anatomischen Körperstruktur erfolgt, die auf medizinischen Analyseverfahren, wie beispielsweise Magnetresonanz-Tomographie (MRT) oder Computer-Tomographie (CT) basieren, wobei diese Analyseverfahren typischerweise zu einem früheren Zeitpunkt insbesondere präoperativ durchgeführt werden und eine detailreiche Information über die Körperstruktur bieten. Diese detailreiche Information wird insbesondere zur Planung einer Operation verwendet. Es wird nun gewünscht, das virtuelle Bezugssystem, genauer die (virtuellen) Daten über die anatomische Körperstruktur (auf denen beispielsweise die Planung einer Operation beruhen), mit insbesondere interoperativ gewonnenen (realen) Körperstrukturdaten abzugleichen. Hierzu sind üblicherweise Markereinrichtungen an den Körperstrukturen des Patienten, beispielsweise am Knochen (z.B. Pelvis und Femur) angebracht, deren Detektion die Gewinnung realer Körperstrukturdaten erlaubt. (Die Begriffe "real" und "virtuell" werden im Folgenden verwendet, um die vorgenannten Bezugssysteme und die entsprechenden Daten und Objekte zu unterscheiden). Die Lagebeziehung zwischen der anatomischen Körperstruktur und den Markereinrichtungen ist jedoch häufig zu Beginn der Operation nicht bekannt. Um dennoch das virtuelle Bezugssystem mit dem realen abzugleichen, werden üblicherweise mittels eines Pointers so genannte Landmarken der Körperstruktur (interoperativ) berührt. An dem Pointer sind ebenfalls Marker angebracht, deren relative Lage zur Spitze des Pointers bekannt ist, so dass die Spitze des Pointers und damit die Lage der Landmarke durch eine Markerdetektionseinrichtung detektiert werden kann. Diese Markerdetektionseinrichtung detektiert auch die an der Körperstruktur angebrachte Markereinrichtung, so dass sich hieraus die relative Lage zwischen der Markereinrichtung und der Spitze des Pointers und damit die relative Lage zwischen der Markereinrichtung und der Landmarke ergibt. Vorzugsweise werden vom Operateur mindestens drei Landmarken mittels des Pointers im realen Bezugssystem erfasst und in ein Navigationssystem eingelesen. Dieselben (korrespondierenden) Landmarken werden dann im virtuellen Bezugssystem vom Arzt identifiziert. Basierend auf den bestimmten Paaren von korrespondierenden Landmarken, die ein Beispiel für Korrespondenz-Objekt-Paare darstellen, kann dann mit einer gewissen Genauigkeit, die auch von der relativen Lage der Landmarken abhängt, die räumliche Beziehung zwischen dem realen und virtuellen Bezugssystem hergestellt werden. In Folge dessen ist es beispielsweise möglich, ein mit einer Markereinrichtung versehenes Instrument relativ zur anatomischen Körperstruktur zu navigieren, wobei die Lage des Instruments relativ zu den virtuellen Körperstrukturdaten beispielsweise an einem Bildschirm beobachtet werden kann.

In der Realität ist die Bestimmung der Landmarken für den Arzt jedoch mit Schwierigkeiten verbunden. Zum einen ist üblicherweise nur ein kleiner Bereich der anatomischen Körperstruktur (z.B. Knochenstruktur) freigelegt, so dass in diesem kleinen Bereich nur wenige markante Oberflächenformen der Körperstruktur vorhanden sind, die als Landmarken verwendet werden können. Anders ausgedrückt ergeben sich größere Unsicherheiten bei der Bestimmung der Landmarken, falls die Oberflächenstruktur des Knochens nicht markant ist.

US 6,096,050 zeigt ein System und ein Verfahren zur punktweisen Korrelation eines realen Körpers mit einem Datensatz des Körpers, der mit einem bildgebenden Verfahren erfasst wurde. Diese Schrift beschäftigt sich insbesondere mit der Ermittlung falsch erkannter geometrischer Symmetrien in den Datensätzen sowie deren Korrektur mithilfe einer Projektion der Datenpunkte auf ein Gradnetz.

WO 2006/106335 A1 zeigt ein Verfahren und ein System zum Registrieren der Position eines Körperteils mit einem computergestützten chirurgischen System. Diese Schrift betrifft insbesondere die Annäherung eines virtuellen Patientendatensatzes an einen real erfassten Patientendatensatz sowie die Ermittlung eines Gütemaßes für die Näherung des virtuellen Datensatzes an den realen Datensatz. Ferner wird eine Hinweisinformation über dieses Gütemaß für wenigstens einen der genäherten Punkten ausgegeben.

Aufgabe der Erfindung ist es den Benutzer des Navigationssystems beim Abgleich des realen und virtuellen Bezugssystems und insbesondere bei der Bestimmung von Korrespondenz-Objekt-Paaren (Korrespondenz-Landmarken-Paaren) zu unterstützen.

Vorstehende Aufgabe wird durch die Gegenstände der unabhängigen Ansprüche gelöst. Die abhängigen Ansprüche sind auf vorteilhafte Ausführungsformen gerichtet.

Wie oben erwähnt, betrifft die vorliegende Erfindung die Bestimmung von Korrespondenz-Objekt-Paaren, die dazu verwendet werden können, um verschiedene Bezugssystem abzugleichen. Diese beiden Bezugssysteme werden im Folgenden A-Bezugssystem und B-Bezugssystem genannt. Das A-Bezugssystem kann beispielsweise das virtuelle Bezugssystem sein und das B-Bezugssystem das reale Bezugssystem. Dies kann aber auch umgekehrt sein, da insbesondere gemäß der Erfindung die Reihenfolge und Abfolge, mit der ein Objekt in einem A-Bezugssystem (als ein A-Objekt) und ein Objekt (B-Objekt) in einem B-Bezugssystem bestimmt wird, beliebig ist, insbesondere alternierend ist und auch mehrere A-Objekte oder B-Objekte hintereinander bestimmt werden können, bevor ein Objekt im jeweils anderen Bezugssystem bestimmt wird. Soweit im Folgenden von einem ersten und zweiten Objekt die Rede ist, wird davon ausgegangen, dass sich die beiden Objekte unterscheiden und verschiedene Teile der Körperstruktur darstellen.

Erfindungsgemäß werden vorzugsweise geometrische Beziehungen zwischen zwei oder mehr Objekten in einem ersten Bezugssystem genutzt, um im anderen zweiten Bezugssystem korrespondierende Objekte basierend auf den geometrischen Beziehungen zwischen den Objekten im ersten Bezugssystem aufzufinden. Dabei wird insbesondere davon ausgegangen, dass die geometrischen Beziehungen zwischen den Objekten in beiden Bezugssystemen verglichen werden können, wenn es sich um korrespondierende Objekte handelt.

Sind beispielsweise (zwei) A-Objekte im A-Bezugssystem festgelegt und (zwei) B-Objekte im B-Bezugssystem festgelegt, so wird erfindungsgemäß die geometrische Beziehung zwischen den Objekten im jeweiligen Bezugssystem basierend auf der Lage der Objekte im jeweiligen Bezugssystem bestimmt (berechnet). Diese geometrischen Beziehungen werden A-Beziehungsdaten für das A-Bezugssystem genannt und B-Beziehungsdaten für das B-Bezugssystem genannt. Die A-Beziehungsdaten und die B-Beziehungsdaten stellen zusammengenommen Vergleichsdaten dar, die den Vergleich zwischen den A-Beziehungsdaten und den B-Beziehungsdaten ermöglichen. Beispielsweise können sowohl die A-Beziehungsdaten als auch die B-Beziehungsdaten angezeigt werden. Beispielsweise kann der Abstand zwisehen zwei A-Objekten und der Abstand zwischen zwei B-Objekten angezeigt werden. Stimmen die Abstände zumindest in etwa überein, so kann der Bediener, insbesondere Arzt davon ausgehen, dass er zumindest mit einer höheren Wahrscheinlichkeit als ohne die erfindungsgemäße Unterstützung die zwei korrespondierenden Objektpaare korrekt bestimmt hat.

Vorzugsweise werden automatisch oder beispielsweise durch ein Eingabemittel ein oder mehrere, vorzugsweise mindestens drei Korrespondenz-Objekt-Paare mit Hilfe der Vergleichsdaten basierend auf den festgelegten A-Objekten und B-Objekten bestimmt. Die automatische Bestimmung der Paare kann beispielsweise basierend auf einem Vergleich der Beziehungsdaten erfolgen, falls beispielsweise die Abweichung zwischen den Beziehungsdaten unterhalb eines vorbestimmten Umfanges liegt. Alternativ oder zusätzlich kann eine Bestimmung von Paaren von korrespondierenden Objekten durch einen Bediener erfolgen, indem er beispielsweise festgelegte Objekte (z.B. ein A-Objekt und ein B-Objekt) als korrespondierende Objekte bestimmt. Das erfindungsgemäße Verfahren ist also nicht nur zur Unterstützung der Bestimmung der Lage vom Korrespondenz-Objekt-Paaren geeignet, sondern auch zu deren Bestimmung. Ist eine noch nicht ausreichende Anzahl von Paaren von korrespondierenden Objekten bestimmt, um die Beziehung zwischen den beiden Bezugssystemen vollständig und ausreichend genau zu bestimmen, so kann dennoch basierend auf den bereits bestimmten korrespondierenden Objekt-Paaren (z.B. basierend auf einem korrespondierenden Objektpaar oder auf zwei korrespondierenden Objektpaaren) die räumliche Beziehung zwischen den beiden Bezugssystemen zumindest teilweise bestimmt werden, sofern vorzugsweise bereits mindestens drei Korrespondenz-Objekt-Paare bestimmt oder vorläufig bestimmt sind. Basierend auf dieser teilweisen Bestimmung (die im folgenden auch Ausrichtung oder teilweise Registrierung genannt wird) der A-Objekte im B-Bezugssystem und/oder der B-Objekte im A-Bezugssystem, werden dann Hinweisinformationen für einen Bediener errechnet, die insbesondere auf Punkten, Linien, Flächen oder abgegrenzten Räumen basieren, die Bereiche für potenzielle Korrespondenz-Objekt-Paare darstellen. Durch die Hinweisinformation wird ein Bediener (z.B. akustisch und/oder visuell) darauf hingewiesen, dass er sich in einem derartigen Bereich befindet oder nicht und/oder wie weit er von einem derartigen Bereich entfernt ist. Eine Ausrichtung der beiden Bezugssysteme basierend auf einem oder mehreren gegebenen Korrespondenz-Objekt-Paaren ermöglicht es also erfindungsgemäß, die Hinweisinformationen zu verfeinern und für einen Bediener verständlicher zu gestalten.

Um die Hinweisinformationen zu verfeinern und weitere Informationen für einen Benutzer erzielen zu können, können alternative oder zusätzlich erfindungsgemäß die A-Objekte, B-Objekte und/oder die später noch erwähnten Hilfsobjekte in einen gemeinsamen Koordinatenraum transformiert oder projiziert werden. Dieser gemeinsame Koordinatenraum ist nicht notwendigerweise ein geometrischer Raum, sondern kann beispielsweise auch ein Merkmalsraum sein. Letzteres ergibt sich beispielsweise, wenn die vorgenannten Objekte einer Fouriertransformation oder einer Formanalyse unterzogen werden, um weitere Vergleichsdaten, insbesondere Vergleichwerte im Koordinatenraum erzielen zu können.

Ist der Bediener mit der Übereinstimmung der Beziehungsdaten nicht zufrieden, so kann er die Lage der Objekte in den jeweiligen Bezugssystemen ändern und/oder durch neue Objekte ersetzen, bis er mit der Übereinstimmung der A-Beziehungsdaten und B-Beziehungsdaten zufrieden ist. Bevorzugt wird ein Bediener dabei diejenigen Objekte ändern bzw. ersetzen, deren Identifikation auf der Körperstruktur als Landmarke schwierig ist und somit mit einer größeren Unsicherheit behaftet ist.

Vorzugsweise werden erfindungsgemäß die Vergleichsdaten bereitgestellt, die die geometrischen A-Beziehungsdaten und die geometrischen B-Beziehungsdaten umfassen und deren Vergleich ermöglichen. Die bereitgestellten Vergleichsdaten werden erfindungsgemäß in weiteren Schritten verwendet. Beispielsweise, um eine Hinweisinformation für einen Bediener zu erstellen, die beispielsweise die beiden A-Beziehungsdaten und B-Beziehungsdaten anzeigt, um beispielsweise in einem nächsten Schritt einen Vergleich der Vergleichsdaten durchzuführen, und das Vergleichsergebnis (Vergleichswert) beispielsweise im Rahmen einer Hinweisinformation anzuzeigen. Die Vergleichsdaten können weiter verwendet werden, um das Ersetzen von A- und/oder B-Objekten basierend auf den Vergleichdaten manuell oder automatisch durchzuführen. Sie werden beispielsweise weiter dazu verwendet, um die Lage von A-und/oder B-Objekten insbesondere automatisch zu ändern oder deren Änderung durch Hinweisinformationen zu führen. Die bereitgestellten Vergleichsdaten werden insbesondere dazu verwendet, um zu überprüfen, ob die festgelegten A-Objekte und/oder B-Objekte sich für ein Korrespondenz-Objekt-Paar eignen und/oder ob festgelegte Objekt-Paare einer Konsistenzprüfung genügen. Vergleichswerte können insbesondere auch so erzielt werden, dass mehrere berechnete Vergleichswerte kombiniert werden oder aus diesen eine Auswahl getroffen wird. Beispielsweise kann eine Kombination aus dem Vergleich der Abstände und aus einem Vergleich von Winkelbeziehungen herangezogen werden, um einen Vergleichswert z.B. durch Multiplikation zweier Vergleichswerte oder als Maximal-, Minimal- oder Mittelwert der Vergleichswerte zu bestimmen, der dann als Grundlage für eine Hinweisinformation für den Bediener genutzt wird oder basierend auf dem automatisch ein Korrespondenz-Objekt-Paar ausgewählt wird. Auch ist es möglich, nur einen Vergleichswert aus verschiedenen auszuwählen, also beispielsweise nur einen Vergleichswert auszuwählen, der auf einer Winkelbeziehung basiert, aber den Vergleichswert nicht auszuwählen, der sich auf einem Abstand bezieht. Dies kann man je nach gegebener Situation festlegen, um so eine möglichst stabilen und verlässlichen Vergleichswert zu erhalten.

Vorzugsweise wird der Bediener bei der Änderung der Lage eines der Objekte (A-Objekt oder B-Objekt) oder beim Ersetzen eines Objekts durch ein neues Objekt durch Hinweisinformationen unterstützt, die auf den Vergleichsdaten basieren. Dabei wird vorzugsweise die Lage der anderen, nicht zu ersetzenden oder nicht zu ändernden Objekte als gegeben angenommen und die Hinweisinformationen geben einen Hinweis darauf, wie weit die A-Beziehungsdaten und die B-Beziehungsdaten übereinstimmen, während der Bediener beispielsweise die Lage eines Objektes (A-Objekt oder B-Objekt) ändert. Beispielsweise kann akustisch darauf hingewiesen werden, wenn sich die beiden Beziehungsdaten in Hinblick auf den Vergleichswert annähern oder weiter voneinander entfernen. Handelt es sich bei dem Objekt um ein virtuelles Objekt, so kann auf einem Bildschirm basierend auf den geometrischen Beziehungsdaten des realen Raums eine Auswahl virtueller Kandidat-Objekte angezeigt werden, die den Beziehungsdaten des realen Raums entsprechen würden. Ist beispielsweise durch zwei Objekte im realen Raum ein bestimmter Abstand (als reale Beziehungsdaten) vorgegeben und ist im virtuellen Raum nur ein Objekt vorgegeben, so liegen Kandidat-Objekte im virtuellen Raum auf der Oberfläche einer Kugel deren Zentrum mit dem bereits bestimmten Objekt im virtuellen Raum übereinstimmt und deren Radius mit dem Abstand der beiden Objekte im realen Raum übereinstimmt. Genauer liegen die Kandidat-Objekte auf Punkten oder entlang einer Linie oder Linien, die sich durch Schnittbildung zwischen Kugeloberfläche und Oberfläche der Körperstruktur ergeben. Die so angegebenen Information (z.B. angezeigte Kugeloberfläche oder in 2D eine Kreislinie oder Schnittpunkte oder Schnittlinien) ist insbesondere dann hilfreich, wenn das festzulegende Objekt auf einer im Wesentlichen eindimensionalen Körperstruktur, also beispielsweise auf einer gratartigen Körperstruktur oder an einer Kante der Körperstruktur liegt und die Kugeloberfläche den Verlauf und die Oberfläche dieses Grates oder der Kante in einem Winkel, möglichst einem rechtwinkligen Winkel schneidet. In diesem Fall ergeben sich idealer Weise nur einzelne, insbesondere punktförmige Kandidat-Objekte zwischen denen der Bediener dann wählen kann. Weiterhin kann dem Benutzer durch eine vorläufige Registrierung zwischen virtuellem und realem Objekt anhand bereits vorhandener Beziehungsdaten die Ausrichtung der Objekte auf einem Bildschirm angezeigt werden. Damit kann während der Änderung der Objekte die Orientierung im Raum und damit auch das Auffinden der Objekte erleichtert werden.

Die vorgenannte Kugel oder Kugeloberfläche stellt ein Beispiel für ein Hilfsobjekt dar, dass verwendet werden kann, um Hinweisinformationen zu generieren. Insbesondere kann die vorgenannte Kugel auf einem Bildschirm angezeigt werden, falls es sich beispielsweise um eine Kugel im virtuellen Raum handelt oder der Bediener kann basierend auf der Kugelform beim Erfassen weiterer Objekte im realen Raum durch Hinweisinformationen geführt werden. Zur Berechnung der Hilfsobjekte kann insbesondere ein automatisches und/oder interaktives Verfahren zur Festlegung bestimmter Objekte, die insbesondere eine vorgegebene Beziehung zur anatomischen Körperstruktur oder zu den festgelegten A-Objekten und/oder B-Objekten haben, verwendet werden. Bei den Objekten kann es sich insbesondere um geometrische Grundkörper, wie Kugel, Quader, Linie, Vieleck usw. handeln. Weiterhin kann es sich bei den Hilfsobjekten um Regionen im virtuellen oder im realen Raum handeln, die beispielsweise dadurch definiert sind, dass der Vergleichswert aller Objekte in der Region in Bezug auf eine Auswahl gegebener Objekte und/oder Körperstrukturen einen bestimmten Wert annimmt oder in einem bestimmten Wertebereich liegt. Dies ist beispielsweise bei der oben angesprochenen Kugeloberfläche der Fall, bei der genau die Region genau die Punkte enthält, die einen festen, aus den Beziehungsdaten berechneten Abstand besitzen. Die Kugeloberfläche kann in der Folge mit der anatomischen Körperstruktur geschnitten werden, um eine weitere Einschränkung der Auswahlpunkte und damit eine spezifischere Konstruktion der Hilfsobjekte zu erreichen.

Hilfsobjekte können beispielsweise durch Einpassen geometrischer Strukturen, wie beispielsweise geometrischer Grundkörper in zumindest Teile der anatomischen Struktur festgelegt werden. Beispielsweise kann an den Grat einer Knochenstruktur (beispielsweise Vorderkante des Acromions) eine Tangente angelegt werden, oder auf die Glenoidoberfläche eine Ebene angepasst werden. Ein weiteres Beispiel wäre die Einpassung einer Kugel in die durch die Genoidoberfläche umschriebenen Vertiefung. Die vorgenannten Vergleichswerte und/oder Vergleichsdaten können auch herangezogen werden, um die Hilfsobjekte zu bestimmen. Insbesondere könne die Objekte so gewählt werden, dass sich für sie die optimalen Vergleichswerte ergeben. Die Vergleichswerte werden dabei beispielsweise so bestimmt, dass die geometrischen Eigenschaften der Hilfsobjekte im A-Bezugssystem und im B-Bezugssystem untereinander verglichen werden. Beispielsweise können in beiden Bezugssystemen je eine Kugel eingepasst werden und die Durchmesser der Kugeln werden dann verglichen. Auch können aus der geometrischen Beziehung zwischen verschiedenen Hilfsobjekten, beispielsweise einer Winkelbeziehung zwischen zwei Ebenen, geometrische Beziehungsdaten zwischen Hilfsobjekten in beiden Bezugssystemen bestimmt werden. Diese geometrischen Beziehungsdaten können kann wiederum verglichen werden, um einen Vergleichswert zu erzielen.

Um Korrespondenz-Objekt-Paare zu bestimmten, kann auf verschiedene Weise vorgegangen werden. Beispielsweise können eine Vielzahl von A-Objekten und B-Objekten festgelegt werden, die je paarweise Kandidaten für Korrespondenz-Objekt-Paare darstellen. Aus dieser Vielzahl von Kandidat-Objekten werden dann vom Bediener oder automatisch insbesondere diejenigen Paare ausgewählt, für die sich die beste Übereinstimmung der geometrischen Beziehungsdaten (A-Beziehungsdaten und B-Beziehungsdaten) ergibt.

Alternativ kann auch schrittweise vorgegangen werden. Dies bedeutet, dass beispielsweise ein Teil der bereits festgelegten A-Objekte und B-Objekte als zumindest vorläufig gegebene Korrespondenz-Objekt-Paare angenommen werden kann. So kann beispielsweise auch nur mit einem einzigen Paar aus einem A-Objekt und einem B-Objekt begonnen werden. Vorzugsweise ist dies ein Paar, das auf einer besonders markanten und leicht bestimmbaren Landmarke beruht. Ausgehend hiervon kann dann in einem der beiden Bezugssysteme ein weiteres Objekt ausgewählt werden. Hierzu kann auch auf Datenbankinformationen zurückgegriffen werden, die Informationen über typische Abstände und Lagebeziehungen zwischen der ersten bestimmten Landmarke und der als zweiten zu bestimmenden Landmarke beinhalten. Beispielsweise kann basierend auf dieser Information der wahrscheinliche Bereich für die zweite Landmarke in einer Anzeige bildlich hervorgehoben werden. Ein Bediener wählt dann die zweite Landmarke aus. Basierend auf der geometrischen Beziehung zwischen der ersten und der zweiten Landmarke (im realen oder virtuellen Raum) wird dann die Bestimmung der zweiten Landmarke im jeweils anderen Raum (virtueller oder realer Raum) durch Hinweisinformationen (Vergleichsdaten) unterstützt. Die nun bestimmten Objekte können als zumindest vorerst gegebene Korrespondenzpaare angenommen werden, um hierauf basierend dann weitere Korrespondenzpaare zu bestimmen.

Sind mehrere Korrespondenzpaare gegeben, vorzugsweise mindestens drei, so werden vorzugsweise Konsistenzüberprüfungen durchgeführt. Mittels dieser Konsistenzüberprüfungen wird überprüft, ob bisher als Korrespondenz-Objekt-Paare (zumindest vorerst) festgelegte Objektpaare aus jeweils einem A-Objekt und einem B-Objekt nicht nur bezüglich einem weiteren Objektpaar eine gute Übereinstimmung der geometrischen Beziehungsdaten aufweisen (gute Übereinstimmung heißt z.B. eine Übereinstimmung in einem vorbestimmten Umfang), sondern auch bezüglich anderer (inzwischen bestimmter) Objektpaare. Sind beispielsweise drei Objektpaare bestimmt und wurde das Objektpaar eins und zwei basierend darauf bestimmt, dass die geometrische Beziehung zwischen dem ersten A-Objekt und dem zweiten A-Objekt mit der geometrischen Beziehung zwischen dem ersten B-Objekt und dem zweiten B-Objekt übereinstimmt und wurde das dritte Objektpaar so bestimmt, dass die geometrische Beziehung zwischen dem zweiten A-Objekt und dem dritten A-Objekt mit der geometrischen Beziehung zwischen dem zweiten B-Objekt und dem dritten B-Objekt übereinstimmt, so kann man dies mit einer Konsistenzüberprüfung überprüfen. Dabei wird die geometrische Beziehung zwischen dem ersten A-Objekt und dem dritten A-Objekt mit der geometrischen Beziehung zwischen dem ersten B-Objekt und dem dritten B-Objekt verglichen. Stellt sich auch hier eine gute Übereinstimmung ein, so kann man die gewonnenen drei Objektpaare als Korrespondenz-Objekt-Paare festlegen. Ist die Übereinstimmung nicht zufrieden stellend oder will man noch weitere Objektpaare bestimmen, so kann man die Objektpaare als Kandidat-Objektpaare auffassen, wobei die einzelnen A-Objekte und B-Objekte noch geändert werden können, um eine bessere Übereinstimmung der geometrischen Beziehungsdaten zu erzielen. Zu diesem Zweck können vorher festgelegte Objekte durch neue Objekte ersetzt werden oder die Lage festgelegter Objekte kann geändert werden. Im virtuellen Raum erfolgt dies beispielsweise durch das Verschieben eines Objekts vom Bildschirm mittels der Maus. Im realen Raum kann dies durch eine Bewegung des Pointers von einem Oberflächenpunkt auf der Körperstruktur zu einem anderen Oberflächenpunkt erfolgen.

Insbesondere ist das Verfahren vorzugsweise so gestaltet, dass ein Bediener automatisch aufgefordert wird, Objekte zu ändern oder neue Objekte einzugeben, die ein zusätzliches Objekt darstellen sollen oder bestehende Objekte ersetzen sollen, wenn ein Vergleich der A-Beziehungsdaten mit den B-Beziehungsdaten (also basierend auf den Vergleichsdaten) nicht zu einem zufrieden stellenden Ergebnis führt oder wenn z.B. durch eine automatische Überprüfung festgestellt wird, dass die Beziehungsdaten vorbestimmte Kriterien nicht erfüllen. Ein derartiges Kriterium ist beispielsweise gegeben, wenn alle bisher festgelegten Objekte im jeweiligen Bezugssystem im Wesentlichen auf einer Gerade liegen. Im Wesentlichen bedeutet hierbei insbesondere, dass sie innerhalb eines vorbestimmten Zylinders liegen, dessen Radius beispielsweise weniger als 30 % oder weiniger als 10 % der Höhe des Zylinders beträgt. In diesem Fall kann davon ausgegangen werden, dass aus den bisher bestimmten Korrespondenz-Objekt-Paaren die räumliche Beziehung zwischen dem virtuellen und realen Raum noch nicht mit einer ausreichenden Sicherheit bestimmt werden kann. Ein anderes Beispiel ist gegeben, wenn die bisher bestimmten Objekte in einem der beiden Räume im Wesentlichen auf einer Ebene liegen. Im Wesentlichen bedeutet hier beispielsweise, dass ein Raum, bei dem die festgelegten Objekte die Ecken bilden, eine vorbestimmte maximale Höhe hat, die beispielsweise weniger als 20 % oder weniger als 10 % des Abstandes zwischen den am weitesten entfernten Objekten beträgt. Alternativ zu den vorgenannten Kriterien kann man beispielsweise basierend auf der Detektionsgenauigkeit der Markereinrichtungen oder der Genauigkeit der Skalierung der Analysedaten, auf denen die virtuell dargestellten Körperstrukturen basieren, weitere absolute Kriterien einführen. Beispielsweise, kann man festlegen, dass die vorgenannte Höhe des beschriebenen Raums oder der vorgenannte Radius des Zylinders eine Mindestgröße aufweist, die z.B. zumindest 5 mm oder mindestens 1 cm beträgt oder ein anderes geeignetes absolutes Kriterium gewählt wird. Insgesamt müssen also ausreichend viele, die räumliche Beziehung der Bezugssysteme betreffende Parameter aus den bestimmten Objekt-paaren bestimmbar sein, um die räumliche Beziehung zwischen den vorgenannten Räumen eindeutig festzulegen. Diese Parameter (z.B. Abstände zwischen Objekten; Winkel zwischen Linien, die Objekte verbinden; Winkel zwischen Ebenen, die durch Objekte gebildet werden etc.) werden auch als Freiheitsgrad bezeichnet. Eine Aufforderung durch das Verfahren, weitere Objekte zu bestimmen, erfolgt also vorzugsweise dann, wenn noch nicht alle die räumliche Beziehung der beiden Bezugssysteme betreffende Freiheitsgrade (mit einer ausreichenden Genauigkeit) bestimmbar sind.

Die vorgenannte Eingabe weiterer Objekte und die Durchführung eines Vergleichs kann insbesondere z. T. automatisch durchgeführt werden. Erfindungsgemäß können automatisch die geometrischen A-Beziehungsdaten und B-Beziehungsdaten verglichen werden. Beispielsweise kann automatisch der Abstand zwischen zwei A-Objekten mit dem Abstand zwischen zwei B-Objekten verglichen werden. Ist eine gute Übereinstimmung gegeben, stimmt also beispielsweise der Abstand mindestens zu einem vorbestimmten Prozentsatz überein, der insbesondere mindestens 90 % oder insbesondere 99 % beträgt und/oder ist die Abweichung kleiner als ein vorbestimmter Absolutbetrag, der z.B. kleiner 1 cm, 5 mm oder 1 mm ist, so wird ein entsprechendes Hinweissignal ausgegeben.

Alternativ oder zusätzlich kann basierend auf dem Vergleichsergebnis die Lage eines Objekts automatisch geändert werden. Sind beispielsweise zwei reale Objekte und zwei virtuelle Objekte vorgegeben, so kann die Lage eines der beiden virtuellen Objekte oder auch beider virtueller Objekte basierend auf dem Vergleichsergebnis automatisch geändert werden, bis sich eine gute oder vollständige Übereinstimmung der Beziehungsdaten ergibt. Bei der Änderung der Lage wird vorzugsweise von den bereits z.B. durch einen Bediener gegebenen (aber mit Ungenauigkeit behafteten) Lagen der virtuellen Objekte ausgegangen. Die Änderung der Lage oder der Lagen kann im vorgenannten Beispiel beispielsweise so erfolgen, dass (automatisch) entlang der Oberfläche der Körperstruktur die Lage eines Objekts so verändert wird, dass es sich näher auf das andere Objekt zu bewegt oder weiter davon weg, je nachdem wie sich eine bessere Übereinstimmung ergibt. Insbesondere kann auch noch eine zusätzliche Zwangsbedingung bei der automatischen Lageänderung eingeführt werden, beispielsweise dahingehend, dass sich das Objekt entlang einer Kante oder einer Gratlinie in der Körperstruktur zur Lageänderung bewegen muss.

Insbesondere wenn dies zu keinem zufrieden stellenden Ergebnis führt, kann der Bediener aufgefordert werden, die Lage des realen oder auch des virtuellen Objekts zu ändern. Insgesamt kann durch eine iterative Vorgehensweise (z.B. durch Änderung der Lage verschiedener realer und/oder virtueller Objekte) die Übereinstimmung der korrespondierenden Beziehungsdaten verbessert werden und insbesondere eine gute Konsistenz erzielt werden. Ist beispielsweise ein gewisses Schema vorgegeben, in welcher Reihenfolge bestimmte Landmarken zu bestimmen sind, so können mit Hilfe des erfindungsgemäßen Systems bei verschiedenen Patienten in sehr vergleichbarer Weise Korrespondenzpaare bestimmt werden, so dass die Vergleichbarkeit von Operationsergebnissen erhöht wird. Dies unterstützt auch die Verlässlichkeit statistisch erhobener Daten über den Operationsverlauf und erhöht die Sicherheit bei der Landmarkenbestimmung für den Arzt.

Die Erfindung erlaubt somit die Optimierung der Erfassung von Objekten, insbesondere von Landmarken und die Optimierung der Definition von Objekten bzw. Landmarken zur (automatischen oder manuellen) Bestimmung von Korrespondenz-Objekt-Paaren. Insbesondere kann, falls als Ausgangspunkt eine sehr genau bestimmbare Landmarke gegeben ist, die Bestimmung anderer Landmarken (Objekte) optimiert werden. Insbesondere ist das Verfahren vorteilhaft, falls Objekte, insbesondere weitere Landmarken auf einer Kante der Oberfläche der Körperstruktur liegen. In diesem Fall gibt es im Wesentlichen nur einen einzigen Freiheitsgrad, der für die Erfassung des Objekts übrig geblieben ist, nämlich entlang der Kante bzw. entlang des Grates. Der Abstand der Objekte ist das Kriterium, um den verbliebenen Freiheitsgrad fest zu legen. Das Objekt muss auf dem Schnittpunkt zwischen der Kante und einer Kugeloberfläche liegen, die die erste Landmarke als Zentrum hat und den Abstand der geplanten Landmarken als Radius. Vorzugsweise wird hierbei darauf geachtet, dass die Orientierung der Kante nicht (ungefähr) gleich der Richtung zwischen den Landmarken (Objekten) ist.

Falls bereits zwei Objekte (Landmarken) erfasst worden sind, kann man wie folgt insbesondere gemäß der vorliegenden Erfindung vorgehen. Die Erfassung der dritten Landmarke kann geführt werden, indem man die Abstände zu den zwei vorhergehend definierten Objekten (Landmarken) zeigt. Der verbliebene Freiheitsgrad kann leicht eingestellt werden, falls das Objekt auf der Oberfläche der Körperstruktur liegen muss. Das Objekt muss am Schnittpunkt der Oberfläche der Körperstruktur und den zwei Kugeloberflächen liegen, wobei die Kugeloberflächen die ersten Landmarken als Zentrum haben und die Abstände der geplanten Landmarken als ihren jeweiligen Radius haben.

Bei vielen praktischen Fällen (z.B. der Registrierung des Pelvis bei Hüftoperationen in lateraler Position, bei der Skapularegistrierung bei Schulteroperationen, bei der Registrierung von Wirbeln bei minimal invasiver Wirbelsäulenoperation), ist es schwierig, mit ausreichender Genauigkeit reproduzierbare Landmarken zu definieren. Die vorliegende Erfindung erlaubt es, ein Verfahren durchzuführen, um mehrere Landmarken zu erzeugen. Dieses Verfahren beruht auf vorerfasster Information (Analysedaten) und auf geometrischen bzw. topologisehen Eigenschaften der Oberfläche der Körperstruktur. Das vorliegende Verfahren stellt eine unterstützende Information oder hilfreiche Führungshinweise für einen Arzt bereit, um diese Landmarken in reproduzierbarer Weise mit der Unterstützung des erfindungsgemäßen Verfahrens zu bestimmen. Der Registrierungsprozess wird robuster und stabiler, wenn man mehrere Landmarken bzw. Objekte verwendet.

Zusätzlich können die Punkte hinsichtlich ihrer Konsistenz überprüft werden. Falls zumindest drei Landmarken erfasst worden sind, kann die Konsistenz der Bestimmung, z.B. der Punktabstand geprüft werden, indem beispielsweise zyklisch von Objekt zu Objekt die Überprüfung durchgeführt wird und Objektlagen gegebenenfalls angepasst werden. Dieses Verfahren zur Überprüfung der Konsistenz kann verbessert werden, falls mehr als drei Objekte durch das erfindungsgemäße Verfahren festgelegt werden. Insbesondere können Objekte neu festgelegt werden oder die Lage der Objekte kann geändert werden. Die Abfolge der Objektfestlegung kann insbesondere einem vorgegebenen Schema folgen, das beispielsweise der Arzt vorher geplant hat.

Das erfindungsgemäße Verfahren unterstützt den Arzt auch bei dem Erlernen der Bestimmung von Landmarken, da dem Arzt eine direkte Rückkopplung darüber gegeben wird, wie gut er die Landmarken festgelegt hat und ob die festgelegte Landmarke mit den bisher festgelegten Landmarken konsistent ist. In der Praxis gab es erhebliche Unterschiede, z.B. 10 bis 15 mm zwischen den erfassten und den geplanten Objekten bzw. Landmarken, falls die Landmarken frei von einem Arzt bestimmt wurden. Das erfindungsgemäße Verfahren erlaubt es, die Genauigkeit der Festlegung von Korrespondenz-Objekt-Paare und insbesondere von Landmarken zu erhöhen. Wie bereits oben erwähnt wird die Erfindung genutzt, um Objekte, insbesondere spezifische Landmarken bei einem Registrierungsprozess, wie er bei der medizinischen Navigation eingesetzt wird, (im realen Raum) zu erfassen oder (im virtuellen Raum) zu definieren. Das System unterstützt den Chirurgen bei der Erfassung bzw. Definition von Objekten, indem bereits verfügbare Information gezeigt wird, und/oder (automatisch) verwendet wird. Die Information kann beispielsweise auf einem Computermonitor angezeigt werden und/oder auch akustisch angegeben werden oder sie kann automatisch bei einem Algorithmus (Programm) zur Definition von Landmarken bzw. Objekten genutzt werden.

Ein Vergleichswert ist insbesondere ein Maß für die Abweichung der zum Vergleich herangezogenen A-Beziehungsdaten und B-Beziehungsdaten voneinander (z.B. Abstand von Punkten, Abstände zu Punkten oder Linien bzw. allgemeine geometrische Distanzen, Winkelabweichungen, Distanz- oder Winkelabweichungen in Projektionen). Der berechnete Wert kann ein beliebiger, von einem Algorithmus berechneter Vergleichswert sein. Dabei können Algorithmen zur Ausrichtung (z.B. Registrierung zwischen A-Objekt und B-Objekt anhand bereits vorliegender Korrespondenzinformationen) und zum Vergleich der Strukturen in einer geeigneten mathematischen Darstellung (z.B. Transformation in ein geeignetes Koordinatensystem ggf. unter Berücksichtigung von Gewichtungen der einzelnen Koordinatenkomponenten) mit verwendet werden.

Zusätzlich kann die Erfindung verwendet werden, um den Arbeitsablauf bei dem Erfassungsprozess zu steuern, indem die Konsistenz der erfassten Objekte basierend auf Kriterien überprüft wird.

Das Kriterium ist gemäß einer Ausführungsform gültig zwischen einigen Paaren oder jedem Paar eines n-Tupels von Objekten. Ein Wert, der die Konsistenz darstellt oder eine visuelle Darstellung der Konsistenz kann beispielsweise auf einem Monitor ausgegeben werden oder z.B. akustisch angezeigt werden. Die oben genannten Vergleichswerte, die insbesondere zwischen verschiedenen Objektpaaren gelten, können mathematisch verarbeitet werden, um einen Wert für eine Konsistenz zu errechnen. Beispielsweise können als Konsistenzwert ein mittlerer Vergleichswert aller bestimmten Vergleichswerte herangezogen werden oder der maximale Vergleichswert, der beispielsweise eine maximale Abweichung von A-Beziehungsdaten von den B-Beziehungsdaten darstellt. Geht man beispielsweise davon aus, dass ein guter Vergleichswert für die Beziehung zwischen dem ersten und zweiten Objektpaar und dem zweiten und dritten Objektpaar gegeben ist, also beispielsweise eine geringe Differenz der Abstände, und das aber zwischen dem ersten und dritten Objektpaar ein schlechter Vergleichswert, also beispielsweise eine große Differenz zwischen den Abständen gegeben ist, so kann dieser maximale Vergleichswert mit der größten Differenz als Kriterium für die Konsistenz, also als Konsistenzwert herangezogen werden. Alternativ kann der Mittelwert der Differenz oder eine andere Kombination der Vergleichswerte, als Konsistenzwert bestimmt werden.

Alternativ oder zusätzlich kann eine neue Erfassung von Objekten automatisch gefordert werden, z.B. falls die Konsistenz automatisch als nicht ausreichend befunden wird oder vom Bediener als nicht ausreichend angesehen wird.

Die Erfassung von Objekten bedeutet hierin insbesondere jegliches Verfahren, um eine Koordinateninformation über Objekte, insbesondere Landmarken zu erhalten. Die Notwendigkeit einer derartigen Erfassung ist insbesondere bei einer interoperativen Situation gegeben. Die Erfassung von Landmarken stellt also insbesondere die Erfassung von Koordinaten relativ zu einer Markereinrichtung (z.B. der Referenzstern, der an einem Knochen befestigt ist) dar. Diese Erfassung kann durch eine jegliche Vorrichtung oder durch ein jegliches Verfahren durchgeführt werden, um eine derartige Lageinformation über die Lage eines Objekts zu erfassen. Beispielsweise kann eine Erfassungseinrichtung, die die vorgenannte Markeinrichtung und beispielsweise einen Pointer oder ein kalibriertes Instrument, insbesondere mit einer daran angebrachten Markereinrichtung, umfasst und die insbesondere eine Detektionseinrichtung zum Detektieren der Markereinrichtung und zum Weiterleiten der Detektionssignale an eine Datenverarbeitungsvorrichtung umfasst, verwendet werden. Auch können Punkte oder Objekte in registrierten Bilddaten (z.B. Fluoroskopiebildern) festgelegt werden. Bilddaten sind dann registriert, wenn den durch sie beschriebenen Bildpunkten eine räumliche Position in einem Bezugssystem zugeordnet werden kann, das insbesondere bezüglich einer an einer Körperstruktur angebrachten (und insbesondere zur Navigation verwendeten) Markereinrichtung ortsfest ist. Die Erfassung von Bilddaten (Fluoroskopiebilder) stellt ein weiteres Beispiel für interoperativ eingesetzte Analyseverfahren dar, mit deren Hilfe reale Objekte im realen Raum relativ zu einer Markereinrichtung bestimmt werden können. Die erfassten Bilddaten werden bevorzugt der erfindungsgemäßen Datenverarbeitungsvorrichtung zugeführt. Die Markereinrichtung wird dabei vorzugsweise ebenfalls von der interoperativ genutzten Analyseeinrichtung erfasst, so dass die Analyseeinrichtung sowohl die Markereinrichtung als auch die Körperstruktur und damit die realen Objekte detektiert, um eine Registrierung zu ermöglichen. Die Erfassungseinrichtung umfasst also in diesem Beispiel die Analyseeinrichtung und die Markereinrichtung. Gemäß einem bevorzugten Verfahren kann auch die Erfassung von Objekten so durchgeführt werden, dass mehrere Objekte erfasst werden und dann die zur Bildung von Korrespondenz-Objekt-Paaren am besten passenden Objekte automatisch ausgewählt werden. Die automatische Auswahl kann beispielsweise auf dem Abstand zwischen den Objekten basieren. Die automatische Auswahl kann sofort oder später, z.B. während eines Registrierungsprozesses durchgeführt werden.

Die Definition von Objekten bzw. Landmarken bedeutet hierin ein jegliches Verfahren, um Information über Landmarken bzw. Objekte in einem gegebenen 3D-Modell einer Körperstruktur oder eines Teils einer Körperstruktur durchzuführen. Die Definition kann automatisch oder manuell/interaktiv erfolgen. Zur automatischen Definition der Landmarken kann die erfasste Information als zusätzlich Zwangsbedingung genutzt werden. Das 3D-Modell kann ein Teil eines dreidimensionalen Bilddatensatzes einer Körperstruktur sein, der beispielsweise mittel CT oder MRT gewonnen wurde oder kann auch jegliche andere dreidimensionale Darstellung des Teils der Körperstruktur sein, also beispielsweise ein statistisches Modell, ein generisches Modell oder ein angepasstes Modell der Körperstruktur oder auch ein dreidimensionales Modell, das von einem Laserscanner beispielsweise erfasst wurde.

Sinn und Zweck von registrierten Bilddaten ist insbesondere die Verwendung der registrierten Bilddatensätze bei navigationsunterstützten Operationen im Bereich der Medizin. Chirurgische Eingriffe, die eine hohe Präzision erfordern, können im Vorfeld präzise geplant und anschließend bildunterstützt durchgeführt werden. Zu diesem Zweck wird im Allgemeinen ein zu navigierendes Behandlungsgerät wie zum Beispiel ein Bohr- oder ein Fräsgerät mit einer so genannten Markereinrichtung versehen. Diese Markereinrichtung am zu navigierenden Behandlungsgerät wird zugleich mit einer weiteren Markereinrichtung, die sich an einer bekannten Position im Raum befindet (z.B. an einer Körperstruktur befestigt ist), durch ein Kamerasystem erfasst. Die relative Lage zwischen der Markereinrichtung am zu navigierenden Behandlungsgerät einerseits und der zweiten Markereinrichtung andererseits wird ermittelt, und daraus wird auf die Position des zu navigierenden Behandlungsgeräts rückgeschlossen. Auf einer Bildschirmeinheit, die der Operateur während der Operation im Blick hat, wird nun ein registrierter Bilddatensatz dargestellt, in dem gleichzeitig die aktuelle Position des zu navigierenden Behandlungsgeräts dargestellt wird. Diese Art der Darstellung setzt voraus, dass den Bilddatenpunkten ebenfalls räumliche Positionen zugeordnet wurden.

Es gibt unterschiedlichste Bilddatensätze, basierend auf denen navigiert werden kann. So ist es grundsätzlich möglich, auf registrierten 2D-Bilddatensätzen basierend zu navigieren (z.B. basierend auf herkömmlichen Röntgenaufnahmen). Des Weiteren ist es möglich, basierend auf registrierten 3D-Bilddatensätzen zu navigieren, wie sie zum Beispiel bei einer Computertomographie oder bei einer Magnetresonanzaufnahme (MRT) erzeugt werden. Bei 3D-Bilddatensätzen werden dem Operateur oftmals Schnittdarstellungen durch verschiedene Ebenen auf einem Bildschirm angezeigt. Die Erfindung erlaubt es virtuelle Körperstrukturdaten, die insbesondere präoperativ gewonnen wurden, durch Abgleich des realen mit dem virtuellen Bezugssystem ebenfalls genau zu registrieren und zur Navigation zu verwenden.

Die folgende detaillierte Ausführungsform offenbart weitere erfinderische Merkmale.
Figur 1a zeigt zwei geplante (virtuelle) Landmarken und Figur 1b zeigt die Erfassung zweier entsprechender realer Landmarken.
Figur 2a und 2b zeigen die Projektion von Objekten in eine Ebene. Figur 2a bezieht sich auf virtuelles Bezugssystem und Figur 2b auf ein reales Bezugssystem.

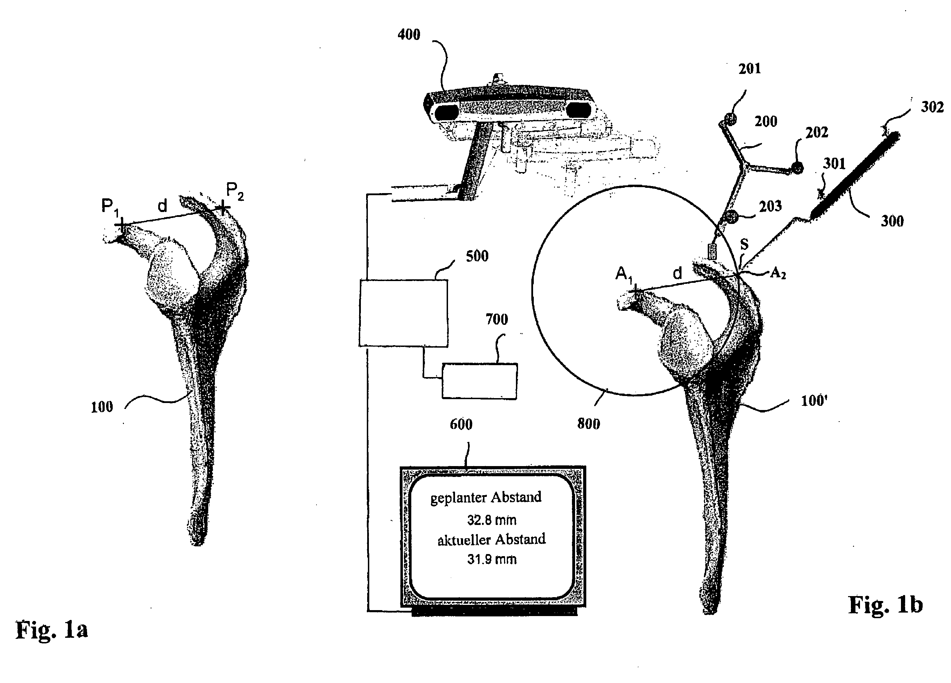

Bei der folgenden Ausführungsform wird beispielsweise davon ausgegangen, dass CT-Daten als virtuelle Daten der Körperstruktur zur Verfügung stehen. Man nehme an, dass erste und zweite Landmarken bereits geplant wurden, also im virtuellen Raum (basierend auf den CT-Daten) festgelegt wurden. Diese ersten und zweiten Landmarken P₁ und P₂ sind in Figur 1a gezeigt und sind mit einer Strecke "d" beabstandet. Da es sich bei einer Registrierung auf CT-Basis um eine starre (rigide) Registrierung handelt, bleiben die Abstände, insbesondere die relativen Abstandbeziehungen erhalten. Somit müssen für die Abstände an der realen Körperstruktur, die insbesondere interoperativ bestimmt werden, für dieselben Landmarken dieselben sein. Die virtuelle Körperstruktur 100 in Figur 1a entspricht der realen Körperstruktur 100' in der Figur 1b. An der realen Körperstruktur 100' ist eine Markereinrichtung 200 (ein Referenzstern) angebracht, die mit Markern (Markerkugeln) 201, 202 und 203 versehen ist, die insbesondere vorbestimmte Lagebeziehungen zueinander haben. Weiter ist ein so genannter Pointer 300, der ebenfalls zwei Markern (Markerkugeln) 301 und 302 aufweist in Figur 1b abgebildet. Die Marker (Markerkugeln) reflektieren oder emittieren Licht (z.B. Infrarotlicht), das von einer Detektionseinrichtung 400 erfasst wird, die mit einer Datenverarbeitungsvorrichtung 500 verbunden ist, die wiederum mit einem Monitor 600 verbunden ist. Die Detektionseinrichtung 400, die Datenverarbeitungsvorrichtung 500 und der Monitor 600 bilden ein erfindungsgemäßes System zur Unterstützung der Bestimmung von Korrespondenz-Objekt-Paaren. Das System kann Bestandteil eines Navigationssystems sein. Die Lagebeziehungen zwischen den Markern (Markerkugeln) sind vorzugsweise in einer Datenbank der Datenverarbeitungsvorrichtung abgespeichert. Insbesondere sind die Lagebeziehungen der Pointerspitze S zu den Markern (Markerkugeln) 301, 302 abgespeichert. Der Referenzstern 200 ist bezüglich der Körperstruktur 100' ortsfest und z.B. eingeschraubt. Bei dem gegebenen Beispiel wird nun angenommen, dass bereits eine Landmarke A₁ im realen Raum an der realen Körperstruktur 100' mittels des Pointers 300 in das erfindungsgemäße System eingelesen wurde bzw. erfasst wurde. Die Lage der Landmarke A₁ relativ zum Referenzstern 200 ist somit dem System bekannt. Dies kann einfach dadurch erfolgen, indem die Spitze des Pointers 300 zum Punkt A₁ geführt wird und mittels einer Eingabeinrichtung 700 (z.B. Maus oder Tastatur) dem System mitgeteilt wird, dass die Spitze S des Pointers eine Landmarke A₁ ist, die mit der geplanten Landmarke P₁ übereinstimmen soll und mit ihr zusammen ein Korrespondenz-Objekt-Paar bilden soll. Aus den geometrischen Beziehungsdaten der geplanten Landmarken P₁ und P₂ ist der Abstand d bekannt. Somit ergeben sich für eine Landmarke A₂, die zu der geplanten Landmarke P₂ korrespondiert eine Vielzahl von möglichen (Kandidaten-)Landmarken, die sich auf der Oberfläche einer Kugel 800 mit dem Radius d befinden. Dem Bediener (Arzt) ist nun bekannt, dass sich die geplante Landmarke P₂ oben auf dem Grat der Knochenstruktur (Vorderkante des Acromions) befinden muss. Weiter wird ihm über den Monitor 600 sowohl der geplante (virtuelle) Abstand d (32,8 mm im Beispiel) als auch der aktuelle (reale) Abstand zwischen Pointerspitze S und A₁ (im Beispiel 31,9 mm) angezeigt. Durch Verfahren der Pointerspitze S entlang des Grates der Knochenstruktur und unter Beachtung der Anzeige auf dem Monitor kann nun der Bediener z.B. mittels der Eingabeeinrichtung einen Punkt als korrespondierendes Objekt A₂ wählen, bei dem der geplante Abstand mit dem aktuellen Abstand möglichst genau übereinstimmt und der zum Zeitpunkt der Wahl identisch mit der Pointerspitze S ist.

Alternativ oder zusätzlich kann beispielsweise auch die Differenz zwischen dem geplanten (virtuellen) Abstand und dem aktuellen (realen) Abstand (Spitze des Pointers) angezeigt werden. Die Spitze S des Pointers stellt somit ein Beispiel für ein Objekt im realen Raum dar. Insbesondere kann man das erfindungsgemäße Verfahren so gestalten, dass die Spitze des Pointers nur dann ein Objekt darstellt, wenn der Pointer die Körperstruktur 100' berührt und/oder wenn eine entsprechende Eingabe z.B. durch eine Eingabeeinrichtung 700 erfolgt und/oder der Bediener Hinweisinformationen z.B. auf dem Monitor 600 wünscht.

Falls der geplante Abstand nicht vollständig mit dem aktuellen Abstand übereinstimmt, kann erfindungsgemäß auch eine automatische Korrektur durchgeführt werden, so dass die Abstände besser oder vollständig übereinstimmen. Hierzu können auch die Objekte (Punkte) P₁ und/oder P₂ verschoben werden.

Wie bereits oben ausgeführt, können natürlich auch erst zwei reale Objekte (Punkte) erfasst werden und dann die zwei entsprechenden geplanten (virtuellen) Objekte (Punkte) definiert werden. Jedenfalls kann natürlich das Erfassen und Definieren von Objekten bzw. Landmarken gemischt werden oder iterativ angewendet werden.

Wie bereits oben ausgeführt kann die Lage erfasster (realer) bzw. definierter (virtueller) Landmarken geändert oder neu justiert werden und zwar automatisch oder unter Führung eines Bedieners. Dies kann alles wiederholt werden, bis z.B. eine ausreichende Konsistenz festgestellt wird.

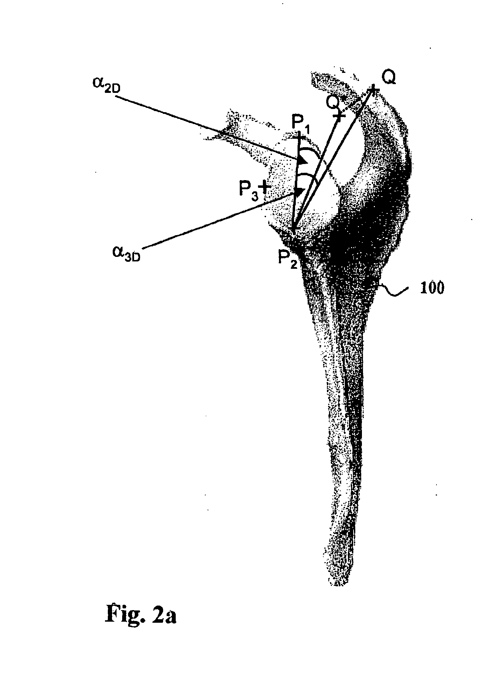

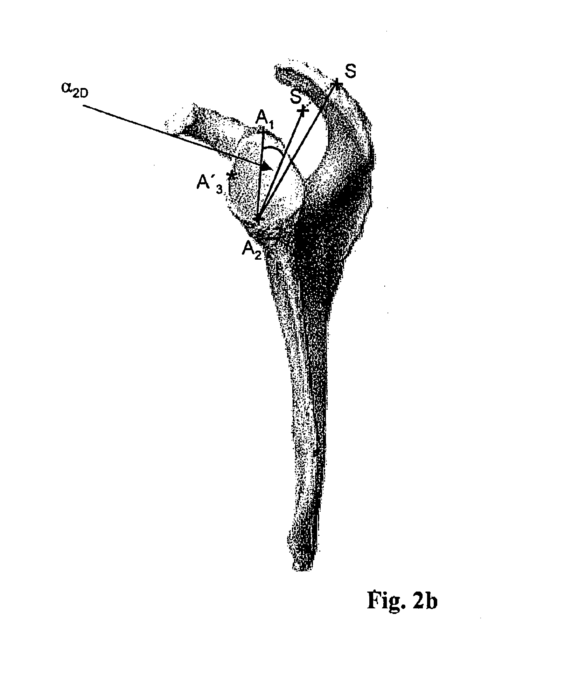

Wie bereits oben ausgeführt, können nicht nur Abstände verwendet werden, sondern beispielsweise auch Winkeldifferenzen, Abweichungen zwischen Ebenen und Projektionen von Landmarken in gewisse Ebenen usw. Ein Beispiel hierfür ist in Figur 2 zu sehen. Die im Folgenden genannten Bezugszeichen zur Bezeichnung von Punkten und Objekten beziehen sich auf die Figuren 2a und 2b. Dort ist die virtuelle Körperstruktur 100 gezeigt. Landmarken P₁, P₂ und P₃ liegen am Rande einer Körperstruktur (Glenoidoberfläche). Sie bilden eine Ebene. Die Landmarke P₃ kann somit auch in Bezug auf die Verbindungslinie zwischen P₁ und P₂ definiert werden. Es kann also als geometrischen Beziehung nicht nur die Beziehung von P₃ zu P₂ oder P₃ zu P₁ bestimmt werden sondern auch die geometrische Beziehung von P₃ zur Verbindungslinie zwischen P₁ und P₂. Beispielsweise kann die Landmarke P₃ auf die Verbindungslinie projiziert werden und der Schnittpunkt der Projektionslinie mit der Verbindungslinie zwischen P₁ und P₂ kann als geometrische Beziehung verwendet werden, um eine Bediener des Verfahrens bei der Bestimmung des realen zu P₃ korrespondierenden Objekts zu leiten.

Wie bereits oben ausgeführt, kann die Genauigkeit beim Abgleich der Bezugssysteme gesteigert werden, wenn nicht alle Punkte in einer Ebene liegen. Deshalb ist es bei dem in Figur 2a gezeigten Beispiel wünschenswert nicht nur Landmarken in einer Ebene, also Landmarken P₁, P₂ und P₃ zu haben, sondern auch noch eine aus dieser Ebene herausragende Landmarke. Diese ist in Figur 2a mit Q bezeichnet. Geometrische Beziehungsdaten können nun z.B. über die Abstände von Q zu einem der Punkte P definiert werden. Sie können aber auch beispielsweise über Winkelbeziehungen definiert werden, die in der Figur 2a mit α_{2D} und α_{3D} bezeichnet sind. α_{3D} stellt einen im Raum liegenden Winkel zwischen der Verbindungslinie von P₁ nach P₂ und von P₂ nach Q dar. Nutzt man diesen Winkel α_{3D} als geometrische Beziehungsdaten, so ist dieser jedoch mit einer größeren Unsicherheit behaftet, als der Winkel α_{2D}, der in der durch die Punkte P₁ und P₂ und P₃ definierten Ebene liegt. (Die Ebene stellt ein Beispiel für ein Hilfsobjekt dar.) Deshalb wird vorzugsweise erfindungsgemäß der Punkt Q in die vorgenannte Ebene projiziert. Dort stellt er den Punkt Q* dar. Diese Winkelbeziehung des virtuellen Punkt Q* in der vorgenannten Ebenen kann dann genutzt werden, um eine zu Q korrespondierende reale Landmarke zu bestimmen. Dabei wird davon ausgegangen, dass bereits zu den Punkte P₁, P₂ und P₃ (Figur 2a) korrespondierende reale Objekte A₁, A₂, A₃ (Figur 2b) bestimmt wurden. Es wird nun die durch die Punkte A₁, A₂ und A₃ definierte Ebene als das zu der durch die Punkte P₁, P₂ und P₃ gegebenen Ebene korrespondierende Hilfsobjekt konstruiert. Die Spitze des Pointers wird bewegt und in die durch die realen Objekte A₁, A₂ und A₃ definierte Ebene projiziert. Falls sich ein dem Winkel α_{2D} entsprechender Winkel im realen Raum einstellt, wird die Lage der Spitze S des Pointers als die Lage des zum virtuellen Objekt Q korrespondierenden realen Objekts bestimmt. Während die Spitze des Pointers bewegt wird kann der reale Winkel angezeigt werden und/oder die Differenz zwischen dem virtuellen Winkel α_{2D} und dem korrespondierenden realen Winkel kann als Führungshinweis für den Bediener angezeigt werden.

Falls das Objekt A₃ im realen Raum noch nicht bestimmt ist, kann alternativ die folgende Ausführungsform verwendet werden. Anhand der Punkte P₁, P₂ und Q im virtuellen Raum sowie A₁, A₂ und S im realen Raum kann eine Ausrichtung (vorläufige Registrierung) der Körperstrukturen vorgenommen werden. In diesem Fall wird die Ausrichtung durch eine möglichst optimale Abbildung der Punkte P₁, P₂ und Q auf A₁, A₂ und S beispielsweise erzielt. Durch diese vorläufige Registrierung kann ein Punkt A'₃ im realen Raum konstruiert werden, für den eine Korrespondenzbeziehung zum Punkt P₃ entsteht. Durch die Punkte P₁, P₂ und P₃ sowie A₁, A₂ und A'₃ können nun entsprechende Ebenen als Hilfsobjekte konstruiert werden, die für die Projektion von Q bzw. S verwendet werden, um Punkte Q* und S* zu bestimmen. Die Differenz der zweidimensionalen Winkel zwischen den Verbindungslinien P₁P₂ und P₂ Q* im virtuellen bzw. A₁A₂ und A₂S* im realen Raum kann nun als Führungsinformation verwendet werden.

## Patentansprüche

1. Verfahren zur Durchführung mittels einer Datenverarbeitungsvorrichtung und zur Unterstützung der Bestimmung der Lage von Korrespondenz-Objekt-Paaren (P₁, A₁; P₂, A_{2;}...), die dieselben Objekte derselben anatomischen Körperstruktur (100, 100') in zwei verschiedenen Bezugssystemen A und B darstellen das folgende Schritte umfasst:
• A-Objekte (P₁, P_{2,} ..., Pₙ; n ≥ 2), die jeweils verschiedene Objekte der Körperstruktur im A-Bezugssystem darstellen, werden festgelegt und B-Objekte (A₁, A_{2,} ..., Aₙ₋₁, S), die jeweils verschiedene Objekte der Körperstruktur im B-Bezugssystem darstellen, werden festgelegt;
• geometrische A-Beziehungsdaten, die eine geometrische Beziehung zwischen den A-Objekten (P₁, P_{2,} ..., Pₙ) beschreiben, werden basierend auf der Lage der A-Objekte (P₁, P_{2,} ..., Pₙ) im A-Bezugsystem bestimmt und geometrische B-Beziehungsdaten, die eine geometrische Beziehung zwischen den B-Objekten (A₁, A_{2,} ..., Aₙ₋₁, S) beschreiben, werden basierend auf der Lage der B-Objekte (A₁, A_{2,} ..., Aₙ₋₁, S) im B-Bezugssystem bestimmt;
• Vergleichsdaten, die die geometrischen A-Beziehungsdaten und die geometrischen B-Beziehungsdaten umfassen und deren Vergleich ermöglichen, werden bereitgestellt und vorzugsweise für eine Anpassung, Änderung, Ersetzung, Korrektur, Überprüfung und/oder automatische Berechnung von A- und/oder B-Objekten verwendet;
• Korrespondenz-Objekt-Paare (P₁, A₁; P₂, A₂) werden basierend auf den festgelegten A-Objekten (P₁, P₂) und B-Objekten (A₁, S) bestimmt,
**dadurch gekennzeichnet, dass**
basierend auf den bestimmten Korrespondenz-Objekt-Paaren (P₁, A₁; P₂, A₂) die räumliche Beziehung zwischen den beiden Bezugssystemen A und B zumindest teilweise bestimmt wird und basierend auf dieser zumindest teilweisen mittels der Datenverarbeitungsvorrichtung durchgeführten Bestimmung der räumlichen Beziehung visuelle und/oder akustische Hinweisinformationen gegeben werden, die auf die mögliche Lage eines Objekts in einem Bezugssystem hinweisen, das zu einem Objekt korrespondiert, das in dem anderen Bezugssystem festgelegt wurde.

2. Verfahren nach Anspruch 1, bei welchem die Lage eines der A-Objekte oder B-Objekte geändert wird oder eines der A-Objekte oder B-Objekte durch ein neues ersetzt wird und die geänderten geometrischen Beziehungsdaten bestimmt werden und die geänderten Vergleichsdaten bereitgestellt werden.

3. Verfahren nach einem der vorhergehenden Ansprüche, bei welchem, wenn ein Vergleich eine Übereinstimmung der geometrischen A-Beziehungsdaten und B-Beziehungsdaten zumindest in einem vorbestimmten Umfang ergibt, eine entsprechende Information ausgegeben und/oder die Festlegung der Lage eines weiteren A-Objekts und/oder B-Objekts durchgeführt wird oder ermöglicht wird oder dazu aufgefordert wird.

4. Verfahren nach einem der vorhergehenden Ansprüche, bei welchem ein Vergleich der geometrischen A-Beziehungsdaten und B-Beziehungsdaten basierend auf den Vergleichsdaten automatisch durchgeführt wird und insbesondere ein Vergleichswert basierend auf den Vergleichsdaten berechnet wird.

5. Verfahren nach einem der vorhergehenden Ansprüche, bei welchem, wenn ein auf den Vergleichsdaten basierender Vergleich keine Übereinstimmung der geometrischen A-Beziehungsdaten und B-Beziehungsdaten zumindest in einem vorbestimmten Umfang ergibt, zumindest einer der folgenden Schritte insbesondere unter Berücksichtigung der Beziehungsdaten durchgeführt wird:
eine entsprechende Information wird ausgegeben;
die Festlegung eines neuen A-Objekts im A-Bezugssystem und/oder B-Objekts im B-Bezugssystem wird ermöglicht, wobei insbesondere das neue A-Objekt ein vorher festgelegtes A-Objekt ersetzt und/oder das neue B-Objekt ein vorher festgelegtes B-Objekt ersetzt;
die Änderung der Lage eines A-Objekts im A-Bezugssystem und/oder die Änderung der Lage eines B-Objekts im B-Bezugssystem wird ermöglicht oder automatisch durchgeführt;
ein vorher festgelegtes A-Objekt wird durch ein neues A-Objekt automatisch ersetzt und/oder ein vorher festgelegtes B-Objekt wird durch ein neues B-Objekt automatisch ersetzt, wobei die Lage des neuen A-Objekts und/oder B-Objekts so bestimmt wird, dass sich beim Vergleich eine bessere Übereinstimmung ergibt als wenn der Vergleich auf dem ersetzen A-Objekt und/oder B-Objekt basiert.

6. Verfahren nach einem der vorhergehenden Ansprüche, bei welchem der Schritt des Ersetzens vorher festgelegter A- oder B-Objekte durch neue A- oder B Objekte und/oder ein Schritt des Änderns der Lage von A- oder B-Objekten wiederholt durchgeführt wird, um die Übereinstimmung zu verbessern.

7. Verfahren nach einem der vorhergehenden Ansprüche, bei welchem eine Vielzahl von A-Objekten und/oder B-Objekten vorgegeben sind und diejenigen Objekte, insbesondere automatisch ausgewählt werden, basierend auf deren Lage sich A-Beziehungsdaten und/oder B-Beziehungsdaten ergeben, für die sich bei einem Vergleich die beste Übereinstimmung ergibt.

8. Verfahren nach einem der bisherigen Ansprüche, bei welchem die geometrischen A-Beziehungsdaten auf der Lage von mehr als zwei A-Objekten basieren und die geometrischen B-Beziehungsdaten auf der Lage von mehr als zwei B-Objekten basieren und bei welchem die Konsistenz der bestimmten Lage der Objekte basierend auf den Vergleichsdaten geprüft wird.

9. Verfahren nach Anspruch 8, bei welchem ein Maß für eine Konsistenz der Lage von bestimmten Korrespondenz-Objekt-Paaren durch einen automatischen Vergleich der Beziehungsdaten und eine automatische Berechnung von Vergleichswerten bestimmt wird.

10. Verfahren nach dem vorhergehenden Anspruch, bei welchem die Hilfsobjekte durch ein Einpassen und/oder Anlegen von geometrischen Strukturen zumindest an Teile der anatomischen Körperstruktur und/oder an Objekte festgelegt werden.

11. Verfahren nach einem der vorhergehenden Ansprüche, bei welchem zur Berechnung des Vergleichswertes Hilfsobjekte bestimmt werden und diese Hilfsobjekte miteinander verglichen oder für weitere Berechnungen verwendet werden.

12. Verfahren nach einem der vorhergehenden Ansprüche, bei welchem der Vergleichswert aus mehreren Vergleichswerten, die auf verschiedenen geometrischen Beziehungen basieren, ausgewählt und/oder kombiniert wird.

13. Verfahren nach einem der vorhergehenden Ansprüche, bei welchem Vergleichswerte zur Bestimmung von Hilfsobjekten verwendet werden.

14. Verfahren nach einem der vorhergehenden Ansprüche, bei welchem für die Bestimmung von Vergleichsdaten, insbesondere für die Berechnung eines Vergleichswertes eine Transformation und/oder Projektion von A-Objekten und B-Objekten und/oder Hilfsobjekten in einen gemeinsamen Koordinatenraum verwendet wird, der einen Vergleich der transformierten Hilfsobjekte und/oder Objekte, die aus unterschiedlichen Bezugssystemen transformiert wurden, ermöglicht, wobei insbesondere Form, Abmessungen und/oder geometrische Beziehungsdaten der Hilfsobjekte und/oder Objekte im Koordinatenraum verglichen werden.

15. Verfahren nach einem der vorhergehenden Ansprüche, bei welchem die Beziehungsdaten zumindest eine der folgenden Beziehungen umfassen:
Abstandsbeziehung zwischen zwei Objekten;
Winkelbeziehungen zwischen den Verbindungslinien, die Objekte verbinden;
Winkelbeziehungen zwischen Ebenen, die durch drei oder mehr Objekte festgelegt werden;
Abstand eines Objekts zu einer Projektion des Objekts auf eine Verbindungslinie und/oder in eine Ebene;
Winkelbeziehungen, die sich durch Projektionen eines Objekts auf eine Verbindungslinie oder eine Ebene ergeben.

16. Verfahren zum Bestimmen der Lage von Korrespondenz-Objekt-Paaren, wobei das Verfahren ein Verfahren nach einem der vorhergehenden Ansprüche umfasst und weiter folgende Schritte umfasst:
Korrespondenz-Objekt-Paare (P₁, A₁; P₂, A₂) werden -basierend auf den Vergleichsdaten bestimmt.

17. Verfahren nach dem vorhergehenden Anspruch, bei welchem eine automatische Ausrichtung zumindest von Teilen der anatomischen Körperstruktur und/oder von einigen der Objekte anhand der bestimmten Korrespondenz-Objekt-Paare vollzogen wird und basierend auf den ausgerichteten Objekten oder Strukturen Vergleichsdaten bestimmt werden und insbesondere zumindest ein Vergleichswert berechnet wird.

18. Programm, das, wenn es auf einem Computer ausgeführt wird oder in einen Computer geladen wird, den Computer veranlasst, das Verfahren nach einem der Ansprüche 1 bis 17 durchzuführen.

19. Datenverarbeitungsvorrichtung (500) mit einem Mittel zur Ausführung des Programms nach Anspruch 18

20. System zur Unterstützung der Bestimmung der Lage von Korrespondenz-Objekt-Paaren, die dieselben Objekte derselben anatomischen Körperstruktur (100, 100') in zwei verschiedenen Bezugssystemen A und B darstellen, um mittels der Korrespondenz-Objekt-Paare (P1, A1) die räumliche Beziehung zwischen zwei Bezugssystemen A und B zu bestimmen, wobei eines der beiden Bezugssysteme als virtuelles Bezugssystem bezeichnet wird und das andere als reales Bezugssystem,
mit der Datenverarbeitungsvorrichtung (500) nach Anspruch 19,
mit einer Datenspeichereinrichtung zum Speichern von auf medizinischen Analyseverfahren basierenden, virtuellen Körperstrukturdaten, die die anatomische Körperstruktur (100) im virtuellen Bezugssystem darstellen, wobei die Datenverarbeitungsvorrichtung ausgebildet ist, basierend auf den virtuellen Körperstrukturdaten die Lage von virtuellen Objekten (P₁, P₂) einer Art der beiden Objektarten, A-Objekt oder B-Objekt, insbesondere mittels eines Eingabemittels (700) festzulegen; und
mit einer Erfassungseinrichtung (200, 300, 400) zum Erfassen der Lage von realen Objekten der anderen Art der beiden Objektarten, A-Objekt oder B-Objekt.

21. System nach Anspruch 20, bei welchem die Datenverarbeitungsvorrichtung (500) ausgebildet ist, basierend auf festgelegten A-Objekten und B-Objekten Korrespondenz-Objekt-Paare (P₁, A₁; P₂, A₂) zu bestimmen.

22. Navigationssystem mit einem System nach Anspruch 21 und mit einem Instrument, an dem eine Instrumenten-Markereinrichtung angebracht ist, deren Lage durch eine Detektionseinrichtung (400) detektierbar ist, die Bestandteil der Erfassungseinrichtung (200, 300, 400) sein kann,
wobei die Datenverarbeitungsvorrichtung (500) ausgebildet ist, die Lage der Instrumenten-Markereinrichtung basierend auf den bestimmten Korrespondenz-Objekt-Paaren im virtuellen Bezugssystem zu bestimmen.

## Claims

1. Method of running, by means of a data processing device, and assisting the operation of locating the position of correspondence-object pairs (P₁, A₁; P₂, A₂; ...) representing the same objects of the same anatomical body structure (100, 100') in two different reference systems A and B,
comprising the following steps:
$ A objects (P₁, P₂, ..., Pₙ; n ≥2) respectively representing different objects of the body structure in the A reference system are set and B objects (A₁, A₂, ..., Aₙ₋₁, S) respectively representing different objects of the body structure in the B reference system are set;
$ geometric A relationship data describing a geometric relationship between the A objects (P₁, P₂, ..., Pₙ) is defined on the basis of the position of the A objects (P₁, P₂, ..., Pₙ) in the A reference system and geometric B relationship data representing a geometric relationship between the B objects (A₁, A₂, ..., Aₙ₋₁, S) is defined on the basis of the position of the B objects (A₁, A₂, ..., Aₙ₋₁, S) in the B reference system;
$ comparison data comprising the geometric A relationship data and the geometric B relationship data and enabling their comparison is made available and preferably used for an adaptation, amendment, replacement, correction, checking and/or automatic calculation of A and/or B objects;
$ correspondence-object pairs (P₁, A₁; P₂, A₂) are defined on the basis of the set A objects (P₁, P₂) and B objects (A_{1,} S),
**characterised in that**
the spatial relationship between the two reference systems A and B is at least partially defined on the basis of the defined correspondence-object pairs (P₁, A₁; P₂, A₂) and, on the basis of this at least partial definition of the spatial relationship by means of the data processing device, visual and/or acoustic reference data indicating the possible position of an object in a reference system corresponding to an object which was set in the other reference system is produced.

2. Method as claimed in claim 1, whereby the position of one of the A objects or B objects is amended or one of the A objects or B objects is replaced by a new one, and the amended geometric relationship data is defined and the amended comparison data made available.

3. Method as claimed in one of the preceding claims, whereby, if a comparison shows a match of the geometric A relationship data and B relationship data at least within a pre-defined range, a message to this effect is output and/or the operation of setting the position of another A object and/or B object is run or made possible or a prompt to run it is output.

4. Method as claimed in one of the preceding claims, whereby the comparison of the geometric A relationship data and B relationship data is run automatically on the basis of the comparison data and in particular a comparative value is calculated on the basis of the comparison data.

5. Method as claimed in one of the preceding claims, whereby, if a comparison based on the comparison data does not result in a match of the geometric A relationship data and B relationship data at least within a pre-defined range, at least one of the following steps is run, in particular taking account of the relationship data:
a message to this effect is output;
the setting of a new A object in the A reference system and/or B object in the B reference system is made possible, and in particular the new A object replaces a previously set A object and/or the new B object replaces a previously set B object;
the amendment to the position of an A object in the A reference system and/or the amendment to the position of a B object in the B reference system is made possible or is applied automatically;
a previously set A object is automatically replaced by a new A object and/or a previously set B object is automatically replaced by a new B object, and the position of the new A object and/or B object is defined so that a better match is obtained during the comparison than it would be if the comparison were based on the replaced A object and/or B object.

6. Method as claimed in one of the preceding claims, whereby the step of replacing the previously set A or B object with new A or B objects and/or a step of amending the position of A or B objects is run on a repeated basis in order to improve the match.

7. Method as claimed in one of the preceding claims, whereby a number of A objects and B objects are predefined and those objects which produce A relationship data and/or B relationship data resulting in the best match during a comparison on the basis of their position are selected, in particular automatically.

8. Method as claimed in one of the preceding claims, whereby the geometric A relationship data is based on the position of more than two A objects and the geometric B relationship data is based on the position of more than two B objects, and whereby the consistency of the defined position of the objects is checked on the basis of the comparison data.

9. Method as claimed in claim 8, whereby a measure of the consistency of the position of defined correspondence-object pairs is determined by an automatic comparison of the relationship data and an automatic calculation of comparison values.

10. Method as claimed in one of the preceding claims, whereby the auxiliary objects are set by fitting and/or placing geometric structures on at least parts of the anatomical body structure and/or on objects.

11. Method as claimed in one of the preceding claims, whereby auxiliary objects are defined with a view to calculating the comparison value and these auxiliary objects are compared with one another or used for other calculations.

12. Method as claimed in one of the preceding claims, whereby the comparison value is selected from and/or combined with several comparison values based on different geometric relationships.

13. Method as claimed in one of the preceding claims, whereby comparison values are used to define auxiliary objects.

14. Method as claimed in one of the preceding claims, whereby a transformation and/or projection of A objects and B objects and/or auxiliary objects in a common co-ordinate space enabling a comparison of the transformed auxiliary objects and/or objects that were transformed from different reference systems are used to define comparison data, in particular to calculate a comparison value, and in particular the shape, dimensions and/or geometric relationship data of the auxiliary objects and/or objects are compared in the co-ordinate space.

15. Method as claimed in one of the preceding claims, whereby the relationship data contains at least one of the following relationships:
distance relationship between two objects;
angular relationships between the connecting lines connecting the objects;
angular relationships between planes defined by three or more objects;
distance of an object from a projection of the object onto a connecting line and/or in a plane;
angular relationships obtained from projections of an object onto a connecting line or a plane.

16. Method of determining the position of correspondence-object pairs, which method is a method as claimed in one of the preceding claims and further comprises the following steps:
correspondence-object pairs (P₁, A₁; P₂, A₂) are defined on the basis of the comparison data.

17. Method as claimed in the preceding claim, whereby an automatic orientation of at least parts of the anatomical body structure and/or of some of the objects is carried out using the defined correspondence-object pairs, and comparison data is determined on the basis of the oriented objects or structures and in particular at least one comparison value is calculated.

18. Programme which, when run on a computer or loaded onto a computer, prompts the computer to run the method as claimed in one of claims 1 to 17.

19. Data processing device (500) with a means for running the programme as claimed in claim 18.

20. System for assisting the operation of defining the position of correspondence-object pairs representing the same objects of the same anatomical body structure (100, 100') in two different reference systems A and B in order to define the spatial relationship between two reference systems A and B by means of the correspondence-object pairs (P1, A1), one of the two reference systems being called a virtual reference system and the other a real reference system,
with the data processing device (500) as claimed in claim 19,
with a data storage device for storing virtual body structure data based on medical test methods which represents the anatomical body structure (100) in the virtual reference system, and the data processing device is configured so that it defines the position of virtual objects (P₁, P₂) of one type of the two object types, A object or B object, in particular by means of an input means (700); and
with a detection system (200, 300, 400) for detecting the position of real objects of the other type of the two object types, A object or B object.

21. System as claimed in claim 20, in which the data processing device (500) is set up to define correspondence-object pairs (P₁, A₁; P₂, A₂) based on set A objects and B objects.

22. Navigation system with a system as claimed in claim 21 and with an instrument to which an instrument marker device is attached, the position of which can be detected by a detection unit (400) which may be a constituent part of the detection system (200, 300, 400),
and the data processing device (500) is set up to determine the position of the instrument marker device based on the defined correspondence-object pairs in the virtual reference system.

## Revendications

1. Procédé pour mettre en oeuvre, au moyen d'un dispositif informatique, et pour assister la détermination de la position de paires d'objets de correspondance (P₁, A₁ ; P₂, A₂ ; ...) qui représentent les mêmes objets de la même structure corporelle anatomique (100, 100') dans deux systèmes de référence A et B différents, qui comprend les étapes suivantes :
• on définit des objets A (P₁, P₂..., Pₙ ; n≥2) qui représentent chacun différents objets de la structure corporelle dans le système de référence A, et on définit des objets B (A₁, A₂..., Aₙ₋₁, S) qui représentent chacun différents objets de la structure corporelle dans le système de référence B ;
• on détermine des données de relation géométrique A, qui décrivent une relation géométrique entre les objets A (P₁, P₂..., Pₙ), sur la base de la position des objets A (P₁, P₂..., Pₙ) dans le système de référence A et on détermine des données de relation géométrique B, qui décrivent une relation géométrique entre les objets B (A₁, A₂, Aₙ₋₁, S), sur la base de la position des objets B (A₁, A₂...Aₙ₋₁, S) dans le système de référence B ;
• on prépare des données comparatives, qui comprennent les données de relation géométrique A et les données de relation géométrique B et qui permettent leur comparaison et on les utilise de préférence pour une adaptation, une modification, un remplacement, une correction, une vérification et/ou un calcul automatique d'objets A et/ou d'objets B ;
• on détermine des paires d'objets de correspondance (P₁, A₁ ; P₂, A₂) sur la base des objets A (P₁, P₂) définis et des objets B (A₁, S) définis,
**caractérisé en ce que**, sur la base des paires d'objets de correspondance (P₁, A₁ ; P₂, A₂) déterminées, on détermine au moins en partie la relation spatiale entre les deux systèmes de référence A et B et, sur la base de cette détermination au moins partielle, exécutée au moyen du dispositif informatique, de la relation spatiale, on obtient des informations indicatives visuelles et/ou acoustiques qui indiquent la position possible d'un objet dans un système de référence qui correspond à un objet lequel a été défini dans l'autre système de référence.

2. Procédé selon la revendication 1, dans lequel la position de l'un des objets A ou des objets B a été modifiée ou l'un des objets A ou des objets B a été remplacé par un nouvel objet et dans lequel on détermine les données de relation géométrique modifiées et on prépare les données comparatives modifiées.

3. Procédé selon l'une quelconque des revendications 1 ou 2, dans lequel, lorsqu'une comparaison indique une concordance des données de relation géométrique A et des données de relation géométrique B au moins dans une mesure prédéterminée, une information correspondante est délivrée et/ou la définition de la position d'un autre objet A et/ou objet B est exécutée, ou rendue possible, est demandée.

4. Procédé selon l'une quelconque des revendications 1 à 3, dans lequel une comparaison des données de relation géométrique A et des données de relation géométrique B est exécutée automatiquement sur la base des données comparatives, et en particulier une valeur comparative est calculée sur la base des données comparatives.

5. Procédé selon l'une quelconque des revendications 1 à 4, dans lequel lorsqu'une comparaison sur la base des données comparatives ne fournit aucune concordance entre les données de relation géométrique A et les données de relation géométrique B, au moins dans une mesure prédéterminée, au moins l'une des étapes suivantes est exécutée, en particulier en tenant compte des données de relation :
une information correspondante est délivrée ;
la définition d'un nouvel objet A dans le système de référence A et/ou d'un objet B dans le système de référence B est rendue possible, le nouvel objet A remplaçant en particulier un objet A défini précédemment et/ou le nouvel objet B remplaçant un objet B défini précédemment ;
le changement de position d'un objet A dans le système de référence A et/ou le changement de position d'un objet B dans le système de référence B est rendu possible ou exécuté automatiquement ;
un objet A défini précédemment est automatiquement remplacé par un nouvel objet A et/ou un objet B défini précédemment est automatiquement remplacé par un nouvel objet B, la position du nouvel objet A et/ou de l'objet B étant déterminée de manière à obtenir lors de la comparaison une meilleure concordance que lorsque la comparaison se base sur l'objet A et/ou l'objet B remplacé.

6. Procédé selon l'une quelconque des revendications 1 à 5, dans lequel l'étape du remplacement des objets A ou B précédemment définis par les nouveaux objets A ou B et/ou une étape du changement de position d'objets A ou B sont exécutées de manière répétée, afin d'améliorer la concordance.

7. Procédé selon l'une quelconque des revendications 1 à 6, dans lequel un grand nombre d'objets A et/ou d'objets B est prédéfini et sont sélectionnés, en particulier automatiquement, les objets pour lesquels on obtient, lors d'une comparaison, la meilleure concordance, dont la position donne les données de relation A et/ou les données de relation B.

8. Procédé selon l'une quelconque des revendications 1 à 7, dans lequel les données de relation géométrique A se basent sur la position de plus de deux objets A et les données de relation géométrique B sur la position de plus de deux objets B, et dans lequel la consistance de la position déterminée des objets est vérifiée sur la base des données comparatives.

9. Procédé selon la revendication 8, dans lequel on détermine une mesure d'une consistance de la position de paires d'objets de correspondance déterminées, par une comparaison automatique des données de relation et un calcul automatique de valeurs comparatives.

10. Procédé selon la revendication 9, dans lequel les objets auxiliaires sont définis par un ajustement et/ou une application de structure géométrique au moins sur des parties de la structure corporelle anatomique et/ou sur des objets.

11. Procédé selon l'une quelconque des revendications 1 à 10, dans lequel on détermine des objets auxiliaires, pour calculer la valeur comparative, et on compare entre eux ces objets auxiliaires ou on les utilise pour d'autres calculs.

12. Procédé selon l'une quelconque des revendications 1 à 11, dans lequel la valeur comparative est sélectionnée et/ou combinée à partir de plusieurs valeurs comparatives qui se basent sur différentes relations géométriques.

13. Procédé selon l'une quelconque des revendications 1 à 12, dans lequel on utilise des valeurs comparatives pour déterminer des objets auxiliaires.

14. Procédé selon l'une quelconque des revendications 1 à 13, dans lequel pour déterminer des données comparatives, en particulier pour calculer une valeur comparative, on utilise une transformation et/ou une projection d'objets A et d'objets B et/ou d'objets auxiliaires dans un espace commun de coordonnées qui permet une comparaison des objets auxiliaires et/ou objets transformés, qui ont été transformés à partir de différents systèmes de référence, en comparant en particulier la forme, la dimension et/ou les données de relation géométrique des objets auxiliaires et/ou objets dans l'espace de coordonnées.

15. Procédé selon l'une quelconque des revendications 1 à 14, dans lequel les données de relation comprennent au moins l'une des relations suivantes :
relation de distance entre deux objets ;
relations angulaires entre les lignes de liaison qui relient les objets ;
relations angulaires entre des plans qui sont définis par trois objets ou plus ;
distance d'un objet par rapport à une projection de l'objet sur une ligne de liaison et/ou dans un plan ;
relations angulaires qui résultent de projections d'un objet sur une ligne de liaison ou un plan.

16. Procédé pour déterminer la position de paires d'objets de correspondance, le procédé comprenant un procédé selon l'une des revendications 1 à 15 et comprenant en outre les étapes suivantes :
on détermine des paires d'objets de correspondance (P₁, A₁ ; P₂, A₂) sur la base des données comparatives.

17. Procédé selon la revendication 16, dans lequel on procède à une orientation automatique au moins de parties de la structure corporelle anatomique et/ou de quelques-uns des objets à l'aide des paires d'objets de correspondance déterminées, et, sur la base des objets ou des structures orientées, on détermine des données comparatives et on calcule en particulier au moins une valeur comparative.

18. Programme qui, lorsqu'il est exécuté dans un ordinateur ou est chargé dans un ordinateur, provoque la mise en oeuvre du procédé selon l'une des revendications 1 à 17.

19. Dispositif informatique (500) avec un moyen pour l'exécution du programme selon la revendication 18.

20. Système pour assister la détermination de la position de paires d'objets de correspondance qui représentent les mêmes objets de la même structure corporelle anatomique 100, 100' dans deux systèmes de référence A et B différents, afin de déterminer, au moyen des paires d'objets de correspondance (P₁, A₁), la relation spatiale entre deux systèmes de référence A et B, l'un des deux systèmes de référence étant désigné par système de référence virtuel et l'autre par système de référence réel,
avec le dispositif informatique 500 selon la revendication 19,
avec un dispositif de mémorisation de données pour mémoriser des données virtuelles de structure corporelle, sur la base de procédés d'analyse médicale, qui représentent la structure corporelle anatomique (100) dans le système de référence virtuel, le dispositif informatique étant conçu pour définir, sur la base des données virtuelles de structure corporelle, la position d'objets virtuels (P₁, P₂)
d'un type des deux types d'objets, objet A ou objet B, en particulier à l'aide d'un moyen de saisie 700 ; et
avec un équipement de détection (200, 300, 400) pour détecter la position d'objets réels de l'autre type des deux types d'objets, objet A ou objet B.

21. Système selon la revendication 20, dans lequel le dispositif informatique (500) est conçu pour déterminer, sur la base d'objets A et d'objets B définis, des paires d'objets de correspondance (P₁, A₁ ; P₂, A₂).

22. Système de navigation avec un système selon la revendication 21 et avec un instrument sur lequel est placé un dispositif de repère d'instrument, dont la position peut être détectée par un dispositif de détection 400 qui peut faire partie intégrante de l'équipement de détection (200, 300, 400).
le dispositif informatique (500) étant conçu pour déterminer la position du dispositif de repère d'instrument, sur la base des paires d'objets de correspondance dans le système de référence virtuel.
